Europäisches Patentamt

European Patent Office

Office européen des brevets

(19)

(11) Publication number: **0 106 292**
**B1**

(12) **EUROPEAN PATENT SPECIFICATION**

(45) Date of publication of patent specification: **09.09.87**

(51) Int. Cl.⁴: **G 10 K 11/00**

(21) Application number: **83110055.7**

(22) Date of filing: **08.10.83**

(54) Ultrasonic sector scanner having fluid replacement capability.

(30) Priority: **18.10.82 US 434826**

(43) Date of publication of application:
**25.04.84 Bulletin 84/17**

(45) Publication of the grant of the patent:
**09.09.87 Bulletin 87/37**

(84) Designated Contracting States:
**DE FR GB NL**

(56) References cited:
**EP-A-0 071 922**
**EP-A-0 089 131**
**US-A-4 181 120**
**US-A-4 237 901**
**US-A-4 316 271**

(73) Proprietor: **GENERAL ELECTRIC COMPANY**
**1 River Road**
**Schenectady New York 12305 (US)**

(72) Inventor: **Thompson, Craig Robert**
**11112 Gingerwood Way**
**Rancho Cordova California 95670 (US)**
Inventor: **Carnes, Ronald Chris**
**124 Redrock Court**
**Folsom California 95630 (US)**
Inventor: **Naumann, Theodore Fleidner, Jr.**
**3400 Paloran Court**
**Shingle Springs California (US)**

(74) Representative: **Voigt, Reinhard, Dipl.-Ing.**
**European Patent Attorney et al**
**Kaiserstrasse 41**
**D-6000 Frankfurt (Main) 1 (DE)**

Courier Press, Leamington Spa, England.

## Description

This invention relates to handheld ultrasonic scanners according to the first part of claims 1 and 2, respectively. Similar structure is known from US—A—4 181 120.

Ultrasonic diagnostic systems are known and commercially available for medical diagnostic purposes. Briefly, such systems utilize sound transducers to transmit ultrasonic waves (e.g. on the order of several megahertz) into a patient and to receive echo signals. In one mode of operation a handheld transducer array is used for transmission and reception of ultrasonic signals. The echo signals are applied to a time gain compensated amplifier to adjust the echo signals for attenuation in passing through the patient. The adjusted signals are then passed through an analog to digital conversion and video processing circuitry and thence to scan converter circuitry for display formatting.

A handheld sector scanner is disclosed in prior, not pre-published application EP—A—71 922. The disclosed ultrasonic sector scanner includes a housing, a shaft rotatably mounted in the housing, and a motor mounted within the housing and coupled to rotate the shaft. A transducer wheel, coupled to the shaft for rotating a plurality of transducers, is positioned in a sealed cavity in one end of the housing for directing ultrasonic energy through ports to a patient and for receiving echo signals from the patient.

An ultrasound beam transmission system is disclosed in prior, not pre-published EP—A—89131.

Transmission and reception of the ultrasound requires that the acoustic paths between the transducers and the ports be a liquid. Thus, the sealed end of the housing in which the transducers are located is liquid filled. However, liquid losses from the sealed cavity occur as a result of fluid migration through the plastic housings. As a result of the liquid loss a pressure differential develops across the sealed housing joints and tends to pull air into the cavity and in the acoustic path between the transducers and ports. Ingested air causes a dramatic degradation of the sector scanner image performance.

It is known from US—A—4,181,120 to provide a side wall of the housing with a lid made of a soft material. The needle of a syringe is pierced through the lid when small quantities of liquid are required to be supplied to the housing for the purpose of maintaining a predetermined liquid pressure within the housing. However, this requires continuous monitoring of the internal pressure of the housing and specific replenishing steps if any liquid loss has been determined. Further the lid of soft material represents an additional area vulnerable upon handling and operation.

An object of the present invention is an improved sector scanner for use in a medical diagnostic system having a fluid filled cavity in which air entry is prevented.

In accordance with one feature of the invention as claimed the bladder is positioned within the fluid filled cavity and has a port which is connected to the ambient atmosphere. Thus, as fluid escapes from the cavity, air enters the bladder and prevents a pressure differential from developing between the cavity and the external atmosphere. The bladder expands automatically to occupy the space from which the fluid escapes without any monitoring and replenishing steps by an operator.

The invention and objects and features thereof will be more readily apparent from the following detailed description and appended claims when taken with the drawing, in which:

Figure 1 is a perspective view of an ultrasonic sector scanner.

Figure 2 is a side view partially in section illustrating the sector scanner of Figure 1 in accordance with one embodiment of the present invention.

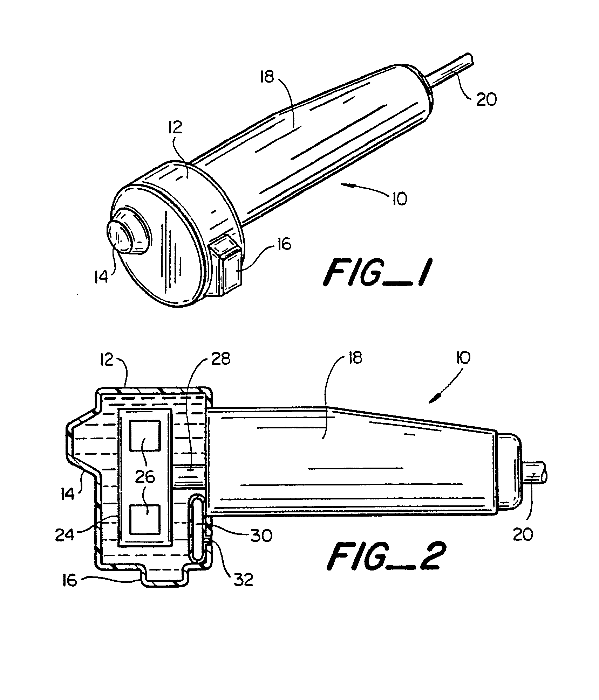

Referring now to the drawings, Figure 1 is a perspective view of an ultrasonic sector scanner which includes a housing shown generally at 10 of suitable configuration for manual support by an operator. The housing 10 is enlarged at one end portion and defines a sealed cavity 12 which accommodates a rotating transducer assembly. Ports 14 and 16 provide for the transmission of ultrasonic energy from the transducers within the sealed cavity 12 and a patient undergoing examination. Signals received by the transducers are passed through internal electronics within the handle portion 18 and through cable 20 to external computer means (not shown) for processing and display. Mounted within handle portion 18 of the housing 10 is a motor and drive shaft which extends into the filled cavity 12 by rotating the transducers.

As above described, the sealed cavity 12 is filled with a fluid to facilitate the flow of ultrasonic energy between the transducers and the ports of the scanner. It has been discovered that over a period of time the fluid will escape from the sealed cavity either through seals in the housing structure or through the housing body, particularly when the housing is made of a plastic material. The loss of fluid creates a differential pressure between the sealed cavity and the ambient atmosphere which results in the entry of air into the sealed cavity. The presence of the air has a degrading effect on the operation of the sector scanner and the electrical signals generated thereby.

In accordance with the present invention a pliable and impermeable bladder is cooperatively arranged with the fluid filled cavity to prevent air entry into the cavity as a result of loss of fluid. Figure 2 is a side view partially in section of the sector scanner of Figure 1 in accordance with one embodiment of the invention. The end portion defining the sealed cavity 12 is shown in section, and mounted therein is the transducer assembly 24 including the transducers 26 which is rotatably mounted on shaft 28. To facilitate illustration of the invention, mirrors are not shown which are normally positioned within the cavity to direct the ultrasonic energy between the transducers 26 and the ports 14 and 16.

Mounted within the fluid filled cavity 12 in the end portion is a pliable and impermeable bladder 30 made of rubber, for example, which has a port 32 extending through the housing wall to the outside atmosphere. When the cavity 12 is initially filled with fluid, the bladder 30 is deflated. Since the bladder 32 is vented to the outside atmosphere, any space due to leakage of the fluid from the cavity 12 will be replaced by inflation of the bladder 30. Thus, space within the cavity previously occupied by the escaping fluid is occupied by the inflating bladder rather than by air entering the cavity. Accordingly, no differential pressure is established between the cavity and the outside atmosphere.

The use of a pliable impermeable bladder to compensate for fluid escaping from the sealed cavity maintains the optimum ultrasonic transmission characteristics of the sector scanner, and the images obtained using signals therefrom do not deteriorate as fluid escapes from the cavity.

**Claims**

1. A sector scanner for use in ultrasonic medical diagnostic systems comprising
   a housing (10) having a sealed end portion,
   a shaft (28) rotatably mounted in said housing,
   a motor mounted within said housing and coupled to rotate said shaft,
   a transducer assembly (24) coupled to said shaft and positioned in said sealed end portion,
   a fluid in said sealed end portion and displacing essentially all air therefrom, and
   a pliable, impermeable bladder means (30) cooperatively arranged with said sealed end portion to prevent air from entering said sealed end portion as a consequence of fluid escaping from said sealed end portion,
   characterized in that
   said bladder means (30) is positioned within said sealed end portion and is vented to the outside atmosphere.

2. A sector scanner having ultrasonic transducer means (24) positioned within a liquid filled, sealed cavity (12), means for compensating for fluid escaping from said sealed cavity comprising a pliable, impermeable bladder means (30) cooperatively arranged with said sealed cavity (12) to prevent air from entering said sealed cavity as a consequence of fluid escaping from said sealed cavity,
   characterized in that
   said bladder means (30) is positioned within said sealed cavity (12) and is vented to the outside atmosphere.

**Patentansprüche**

1. Sektorscanner für medizinische Ultraschall-Diagnosesysteme enthaltend

ein Gehäuse (10) mit einem gekapselten Endabschnitt,
eine Welle (28), die in dem Gehäuse drehbar angebracht ist,
einen Motor, der in dem Gehäuse angebracht und zum Drehen der Welle verbunden ist,
eine Wandlereinrichtung (24), die mit der Welle gekoppelt und in dem gekapselten Endabschnitt angeordnet ist,
ein Fluid in dem gekapselten Endabschnitt, das im wesentlichen die gesamte Luft daraus verdrängt, und
eine biegbare, undurchlässige Blasenvorrichtung (30), die in Zusammenwirkung mit dem gekapselten Endabschnitt angeordnet ist, um zu verhindern, daß Luft in den gekapselten Endabschnitt eintritt als eine Folge eines Fluidaustrittes aus dem gekapselten Endabschnitt,
dadurch gekennzeichnet, daß
die Blasenvorrichtung (30) in dem gekapselten Endabschnitt angeordnet ist und zur Außenatmosphäre entlüftet ist.

2. Sektorscanner mit einer Ultraschall-Wandlereinrichtung (24), die in einer flüssigkeitsgefüllten, gekapselten Kammer (12) angeordnet ist, Mitteln zum Kompensieren von Fluid, das aus der gekapselten Kammer austritt, enthaltend eine biegbare, undurchlässige Blasenvorrichtung (30), die in Zusammenwirkung mit der gekapselten Kammer (12) angeordnet ist, um zu verhindern, daß Luft in die gekapselte Kammer eintritt als eine Folge eines Fluidaustrittes aus der gekapselten Kammer,
dadurch gekennzeichnet, daß
die Blasenvorrichtung (30) in der gekapselten Kammer (12) angeordnet ist und zur Außenatmosphäre entlüftet ist.

**Revendications**

1. Dispositif de balayage de secteur pour emploi dans des systèmes ultrasoniques de diagnostic médical comprenant:
   un logement (10) ayant une partie d'extrémité étanche,
   un arbre (28) monté de manière à pouvoir tourner dans le logement,
   un moteur monté à l'intérieur du logement et accouplé de manière à animer l'arbre d'un mouvement de rotation,
   un ensemble (24) à transducteurs couplé à l'arbre et placé dans la partie d'extrémité étanche,
   un fluide dans la partie d'extrémité étanche et déplaçant essentiellement la totalité de l'air à partir de celle-ci, et
   un moyen de vessie pliable, imperméable (30) disposé de manière à coopérer avec la partie d'extrémité étanche afin d'éviter que l'air n'entre dans la partie d'extrémité étanche par suite d'une fuite de fluide à partir de la partie d'extrémité étanche,
   caractérisé en ce que:
   le moyen de vessie (30) est placé à l'intérieur de la partie d'extrémité étanche et débouche dans l'atmosphère extérieure.

2. Dispositif de balayage de secteur comportant un moyen de transducteurs ultrasoniques (24) placé à l'intérieure d'une cavité étanche, remplie de fluide (12), un moyen pour compenser les fuites de fluide à partir de la cavité étanche comprenant un moyen de vessie pliable, imperméable (30) disposé de manière à coopérer avec la cavité étanche (12) pour éviter que l'air ne s'échappe de la cavité étanche à la suite d'une fuite de fluide à partir de la cavité étanche, caractérisé en ce que:

le moyen de vessie (30) est placé à l'intérieur de la cavité étanche (12) et débouche dans l'atmosphère extérieure.

0 106 292

FIG_1

FIG_2